# EUROPEAN PATENT SPECIFICATION

(11) **EP 2 923 582 B1**
(45) Date of publication and mention of the grant of the patent: **21.03.2018**
(21) Application number: 15164858.1
(22) Date of filing: 14.07.2010
(51) Int. Cl.: A23K 20/00, A23K 50/40, A61K 31/385, A61P 39/06

(54) **PET FOOD COMPOSITIONS INCLUDING SUSTAINED-RELEASE LIPOIC ACID**
HAUSTIERFUTTERZUSAMMENSETZUNG MIT LIPONSÄURE MIT VERZÖGERTER FREISETZUNG
COMPOSITIONS ALIMENTAIRES POUR ANIMAUX DE COMPAGNIE COMPRENANT DE L'ACIDE LIPOÏQUE À LIBÉRATION PROLONGÉE

(30) Priority: 14.07.2009 US 225328 P
(43) Date of publication of application: 30.09.2015
(62) Divisional of application: 10734871.6
(73) Proprietor: Hill's Pet Nutrition, Inc., Topeka, KS 66603 (US)
(72) Inventor: Zicker, Steven C., Lawrence, KS Kansas 66049 (US); Gross, Kathy Lynn, Topeka, KS Kansas 66618 (US)
(74) Representative: Wichmann, Hendrik

(56) References cited:
- WO-A1-2010/083409
- JP-A- 2006 306 825
- US-A1- 2003 044 466
- US-A1- 2003 175 326
- US-A1- 2009 176 864
- US-B2- 6 572 888

## Description

### FIELD OF THE INVENTION

The invention generally relates to pet food compositions that deliver a slow, sustained-release amount of lipoic acid or a salt thereof when fed orally to a companion animal. Also provided are methods of use of the compositions.

### BACKGROUND OF THE INVENTION

Lipoic acid has benefits to companion animals. For example, lipoic acid is important for growth and aging, helps to prevent cell damage, and helps the body rid itself of harmful substances. Natural occurring α-lipoic acid is found as lipoyl-lysine, R-alpha enantiomer, which is believed to undergo minimal cleavage prior to gastrointestinal absorption. In addition, de novo synthesis of α-lipoic acid from fatty acids and cysteine indicate that α-lipoic acid is synthesized in a protein bound form. Bioavailability of α-lipoic acid has been demonstrated to show enantiomeric differences in humans. The bioavailability of lipoyl-lysine has not been evaluated and may be different than free α-lipoic acid. It is well known that during processing of nutrients in commercial pet foods adducts of compounds may occur and impact bioavailability as seen with Maillard products and lysine. Administration of lipoic acid as a capsule with a meal has been evaluated and may impair absorption 10-20%. However, bioavailability of dl-alpha lipoic acid when added prior to extrusion of a pet food has not ever been evaluated and is not known.

US2003175236 discloses a method of treating obesity, a method for reducing the absorption of dietary fat, and a method for treating hypertriglyceridemia in a patient.

US2009176864 discloses compositions and methods for improving animal health.

US2001028896 discloses a controlled release formulation of lipoic acid.

US2003044466 discloses the administration of drugs intended for absorption in the stomach or upper intestinal tract in oral drug delivery systems in conjunction with any of various substances that are stated to function as potent agents for inducing the fed mode.

### SUMMARY OF THE INVENTION

The inventors have developed pet food compositions having a slow-sustained release of lipoic acid when incorporated into a dry expanded pet food kibble. Compared to a single lipoic acid dose in capsule form, lipoic acid incorporated into pet food compositions of the invention results in a lower spike in blood levels of lipoic acid and sustains blood levels of lipoic acid over a longer period.

Accordingly, the inventors have developed pet food formulations including lipoic acid, which is an easy and convenient way for pet owners to dose the lipoic acid, so as to avoid large spikes in blood levels of lipoic acid that could be associated with side effects, and allows the lipoic acid to be delivered by sustained release to cells and tissues following the ingestion of the pet food formulation.

The invention provides a pet food composition according to claim 1. Described is a dried pet food composition having a food component wherein the food component is in the form of a kibble comprising a gelatinized starch matrix and comprises a slow, sustained-release amount of lipoic acid.

It has been found that lipoic acid remains bioavailable and can be delivered to pets over a slow sustained period of time when formulated into a coating on, distributed throughout, or filling in a dried pet food composition. Therefore, the invention offers the advantage of a ready-to-eat pet food composition, which is highly palatable and which contains a shelf stable source of lipoic acid delivered to a companion animal over a slow sustained release period to increase safety and efficacy of the lipoic acid administration.

Described herein is a process of preparing a dried, ready-to-eat pet food composition, the process including cooking a starch source to form a gelatinized starch matrix and forming the gelatinized starch matrix into pieces and drying the pieces, which contain lipoic acid such that the lipoic acid is delivered to pets over a slow, sustained period of time.

In another embodiment, the gelatinized starch matrix is formed into pieces and dried by extruding the gelatinized matrix to form a cooked extrudate and cutting and drying the cooked extrudate to form dried pieces, wherein the pieces include lipoic acid such that the lipoic acid is delivered to pets over a slow sustained period of time. The gelatinized matrix may be caused to expand upon extrusion to form, after cutting and drying, expanded pieces. In another embodiment, the gelatinized starch matrix may be formed into pieces and dried by roller drying the gelatinized starch matrix to form flakes.

In a further embodiment, the gelatinized starch matrix may be formed into pieces and dried by extruding the gelatinized matrix to form a cooked extrudate containing an aperture wherein the aperture includes a sustained-release material and lipoic acid such that the lipoic acid is delivered to pets over a slow sustained period of time; and cutting and drying the pieces.

Also described is a process of preparing a dried, ready-to-eat pet food composition, the process including cooking a starch source to form a gelatinized starch matrix; forming the gelatinized starch matrix into pieces and drying the pieces; and coating or filling the pieces with a substrate which contains lipoic acid such that the lipoic acid is delivered to pets over a slow sustained period of time.

### DETAILED DESCRIPTION

The present disclosure generally encompasses a pet food composition comprising a food component comprising (i) one or more pet food nutrient(s) and (ii) lipoic acid or a salt thereof, which allows a slow, sustained-release amount of lipoic acid. As used herein, the term "lipoic acid" includes lipoic acid and salts thereof. The pet food is in the form of a kibble comprising a gelatinized starch matrix comprising lipoic acid or a salt thereof, which has a sustained-release coating, wherein the sustained-release coating is selected from carnauba wax, spermaceti wax, candellila wax, cocoa butter, cetostearyl alcohol, beeswax, partially hydrogenated vegetable oils, ceresin, paraffin, myristyl alcohol, stearyl alcohol, cetyl alcohol, stearic acid, acacia gum, gelatin, tragacanth, veegum, xanthan gum, carboxymethyl cellulose, hydroxypropyl cellulose, and hydroxyethyl cellulose.

The pet food composition comprises a sustained-release material which is applied to said pet food composition, so as to allow the sustained release of lipoic acid. The sustained-release material may be selected from carboxymethyl cellulose (CMC), hydroxy propyl cellulose (HPC), hydroxy ethyl cellulose (HEC), and combinations thereof.

In various embodiments, the sustained-release material is present in the compositions in amounts of about 4 to about 20 wt %, or about 6 to about 16 wt % or about 8 to about 12 wt %.

In certain embodiments, the lipoic acid is present in an amount of about 1 ppm to about 4500 ppm. In certain embodiments, the lipoic acid is present in an amount of about 10 ppm to about 2500 ppm. In certain embodiments, the lipoic acid is present in an amount of about 50 ppm to about 1500 ppm. In certain embodiments, the lipoic acid is present in an amount of about 100 ppm to about 1000 ppm. In certain embodiments, the lipoic acid is present in an amount of about 200 ppm.

In certain embodiments, the pet is a companion animal such as a dog or cat.

The food composition is in the form of a kibble.

In certain embodiments, the lipoic acid is included in a coating or filling.

In certain embodiments, the gelatinized starch matrix is an extrusion cooked starch source.

In certain embodiments, the coating comprises a carrier substrate, which contains the lipoic acid.

In certain embodiments, the carrier substrate is at least one carrier chosen from the group consisting of a fat, a protein digest, milk solids, a sugar and a particulate flavoring agent.

In certain embodiments, the pet food kibble further includes a source of soluble fiber.

In certain embodiments, the coating comprises a carrier substrate, which contains the lipoic acid.

In certain embodiments, the carrier substrate is a fat, a protein digest, or a mixture thereof.

In certain embodiments, the kibble further includes a source of soluble fiber.

Described herein is a process of preparing a pet food composition, the process comprising cooking a starch source and a protein source to form a gelatinized starch matrix containing protein; adding lipoic acid; forming the gelatinized matrix into kibbles; and drying the kibbles.

In certain embodiments, the starch source and protein source are extrusion cooked; extruded through an orifice; and then cut into kibbles.

Also described herein is a process of preparing a pet food composition, the process comprising cooking a starch source, a protein source, lipoic acid, and a sustained-release material to form a gelatinized starch matrix containing protein and lipoic acid; forming the gelatinized matrix into kibbles; and drying the kibbles.

In certain embodiments, the starch source and protein source are extrusion cooked; extruded through an orifice; and then cut into kibbles.

In another embodiment, the disclosure encompasses pet food kibble comprising:
(i) a gelatinized starch matrix which includes a component chosen from grains such as corn, rice, wheat, beets, barley, oats, soy, and combinations thereof; and
(ii) lipoic acid or a salt thereof.

Described herein are methods for promoting growth, preventing cell damage and/or helping rid the body of harmful substances in an animal in need thereof including administering to an animal a pet food composition comprising a food component, wherein the food component as described above comprises a slow, sustained-release amount of lipoic acid to the animal.

As used herein, the term "nutrient" or "pet food nutrient" refers to a portion of the food composition, which can include up to about 100% of any particular food ingredient suitable for consumption by a companion animal or can include a mixture of food ingredients in various proportions. In certain embodiments, the pet food nutrient includes a combination of food ingredients in amounts of about 0 wt. % to about 50 wt. % fat, about 0 wt. % to about 75 wt. % carbohydrate, about 0 wt. % to about 95 wt. % protein, about 0 wt. % to about 40 wt. % dietary fiber, and about 0 wt. % to about 15 wt. % of one or more nutritional balancing agents.

The quantities administered in the diet, all as wt % (dry matter basis) of the diet, are calculated as the active material that is measured as free material.

The inventors have succeeded in developing a slow, sustained-release lipoic acid pet food composition. By using the composition of the invention in companion pets, it can be shown that an amount of lipoic acid can be delivered in a slow manner and can be maintained in the system for a longer period.

Lipoic acid or alpha-lipoic acid can be administered into the diet as alpha-lipoic acid or as a lipoate derivative as in U.S. Pat. No. 5,621,117, racemic mixtures, salts, esters or amides thereof. The quantity of alpha-lipoic acid can vary according to the age of the pet or the condition being treated. In certain embodiments, the amount of lipoic acid is about 25 ppm, about 50 ppm, about 100 ppm, about 200 ppm, about 500 ppm, about 1000 ppm, about 1500 ppm, about 2000 ppm, about 2500 ppm, about 3000 ppm, about 3500 ppm, about 4000 ppm, about 4500 ppm or about 5000 ppm. In various embodiments, the range of lipoic acid that can be administered to dogs is about 50 ppm to about 4500 ppm. In various embodiments, the range of lipoic acid that can be administered to cats is about 65 ppm to about 2600 ppm. Maximum quantities can vary from about 10 ppm to an amount which remains nontoxic to the pet.

In certain embodiments, the disclosure provides includes a dry pet food containing less than 15% moisture having a porous texture and appearance with fibrous food simulating pieces having a tough, pliable texture interspersed therein.

In another embodiment, the pet food compositions of the disclosure may be produced from any suitable ingredients such as those commonly used in dried, ready-to-eat pet food products. One of these ingredients is a starch source. Suitable starch sources are, for example, grain flours such as corn, rice, wheat, barley, soy and oats. In addition, mixtures of these flours may be used. The flours may be whole flours or may be flours which have had fractions removed; for example, the germ fraction or husk fraction may be removed. Rice flour, corn flour and wheat flour are particularly suitable; either alone or in combination. The starch source will be chosen largely based on the nutritional value, palatability considerations, and the type of product desired.

In certain embodiments, the pet food composition may also contain a protein source. Suitable protein sources may be selected from any suitable animal or vegetable protein source; for example poultry meal, poultry by-product meal, chicken meal, chicken by-product meal, lamb meal, meat and bone meal, fish meal, soy bean meal, soy protein concentrates, milk proteins, corn gluten meal, wheat gluten, gluten and the like. The choice of the protein source will be largely determined by the nutritional needs, palatability considerations and the type of product desired. The starch source may also be a source of protein. In certain embodiments, the protein can be hydrolyzed or a protein isolate.

In certain embodiments, the disclosure encompasses a sustained-release material to allow the sustained release of lipoic acid. In various embodiments, the sustained-release material is present in the compositions in amounts of about 4 to about 20 wt %, or about 6 to about 16 wt % or about 8 to about 12 wt %.

According to the present invention, the lipoic acid is incorporated into or onto a pet food kibble and a timed release or sustained-release coating is applied thereto. For example, the lipoic acid may be contained within or on a substrate as follows: (i) incorporated into matrix spheroids (*e.g*., together with an acceptable spheronizing agent such as cellulose), (ii) incorporated into a normal release core; or (iii) incorporated into a core which comprises a matrix including a sustained-release material. Thereafter, a sustained-release coating is applied onto substrates such as those mentioned in (i)-(iii) above. The pet food compositions including lipoic acid are coated with one or more materials specified above suitable for the regulation of release or for the protection of the formulation. In one embodiment, coatings are provided to permit either pH-dependent or pH-independent release, for example, when exposed to gastrointestinal fluid.

In certain embodiments, the pet food compositions include a combination of one or more material(s) included with the pet food nutrients and lipoic acid, which is formed into a pellet or kibble, and which relies on diffusion or erosion to control release of the lipoic acid. For example, heterogeneous dispersions or solutions of lipoic acid and nutritional agents in water-swellable hydrogel matrices are useful in controlling the release of the lipoic acid by slow surface-to-center swelling of the matrix and subsequent release of the lipoic acid by a combination of diffusion of the nutrient and lipoic acid from the water-swollen part of the matrix and erosion of the water-swollen matrix containing the lipoic acid.

In certain embodiments, the pet food compositions include a sustained-release material, which provides for a sustained release of a lipoic acid according to a desired release profile through the use of one or more of the sustained-release ingredients described herein. In other embodiments, the sustained-release matrix system will provide a release profile, which releases lipoic acid at a substantially constant rate over a designated time period.

The controlled or sustained-release coating contains conventional waxes and waxy materials used in pharmaceutical formulations, selected from carnauba wax, spermaceti wax, candellila wax, cocoa butter, cetostearyl alcohol, beeswax, partially hydrogenated vegetable oils, ceresin, paraffin, myristyl alcohol, stearyl alcohol, cetyl alcohol and stearic acid. Hydrophilic gums are also contemplated for use, in amounts, which can have an effect on the release profile. These hydrophilic gums are selected from acacia, gelatin, tragacanth, veegum, xanthan gum, carboxymethyl cellulose (CMC), hydroxy propyl cellulose (HPC) and hydroxy ethyl cellulose (HEC).

### Methods Of Making The Compositions Of The Disclosure

The pet food composition fortified with lipoic acid may be produced in many different ways.

In one embodiment, the slow, sustained-release pet food compositions of the disclosure are made by an extrusion process, wherein the lipoic acid and sustained-release material is incorporated in the pet food composition by co-manufacturing in the process of a complete extruded food meal.

The process involves blending a mixture to form a premix, cooking and extruding the mixture to form food pieces containing lipoic acid. The mixture is cooked and extruded to yield an expanded dry pet food composition having a porous texture and appearance containing food pieces having a tough, pliable fibrous texture. The respective proportions of the fibrous food pieces and basal matrix provide a food product, which has a uniform mixture that retains its particle integrity during production and upon storage.

The term "dry pet food" or "dry pet food material" is defined as one that has a moisture content less than 15% by weight and typically about 10%. All percentages referred to are understood to be by weight unless specified otherwise and are based upon the weight of the final product.

Conventionally, the dry pet food composition of the disclosure contains crude protein, crude fat, crude fiber, lipoic acid and other minerals and additives. Typical protein components include meat and bone meal and vegetable protein sources such as soybean meal. Other components are also suitable for use in this pet food product.

In still another variation, a portion of the lipoic acid is added with the other dry ingredients that are admixed with water, cooked and worked to form a partially fortified dough. Then, the balance of the lipoic acid can be admixed with the mixture to prepare a lipoic acid containing mixture fortified to desired levels.

The methods further comprise the step of forming the mixture into individual pieces of desirable shape and size, these pieces being in the form of kibbles. Conventional techniques and equipment can be employed to practice this step and the skilled artisan will have no difficulty in selecting those suitable for use herein.

The methods further include the step of drying the shaped and sized individual pieces to form finished dry pet food compositions fortified with lipoic acid.

The skilled artisan will appreciate that the drying step depends in part upon the desired product.

In still another variation, the dry pet food composition containing lipoic acid can be extruded under conditions of temperature and pressure to puff and expand (the "direct expansion" technique) and sectioned or cut into individual pieces to form individual expansions. In this variation, the forming and drying steps are practiced simultaneously rather than sequentially.

In another variation, the drying step can involve heating the pieces under conditions that not only dry the piece but also cause the piece to expand to form dried and puffed or flaked finished pieces. For example, pellets can be gun puffed to form dried puffed pet food composition products.

The finished lipoic acid fortified dry pet food compositions, however formed, can optionally be provided with a topical coating and subsequently finish dried to remove the added moisture from the coating solution to form a finished dry pet food composition. In other variations, a topical coating optionally with salt and/or flavors is applied to form finished dried pet food compositions.

In commercial practice, one or more of the method steps can be combined and performed in or by a single piece of equipment. For example, a dry mix of ingredients including lipoic acid can be admixed with water and/or steam in a cooker extruder such as a single screw or twin screw. The cooker extruder heats, cooks and works the ingredients to form a lipoic acid containing dry pet food composition. In one variation, referred to in the art as direct expansion, the extruder conditions are such that upon extrusion, the cooked product expands and dries and is severed into small pieces. The pieces can be in final form. In slight variations, the pieces can be further dried to final moisture contents.

The dry pet food compositions so prepared can then be conventionally packaged for distribution and sale.

In another embodiment, the dry pet food compositions fortified with lipoic acid can be consumed by a companion animal to obtain the nutritional and physiological benefits of a high lipoic acid diet. A surprising advantage of the dry pet food composition is that the lipoic acid is nearly "invisible," that is, even high levels of lipoic acid are barely organoleptically discernible in the finished product. Surprisingly, the finished dry pet food composition products fortified with lipoic acid are similar to their unfortified counterparts, notwithstanding the presence of the added lipoic acid ingredient. The products are characterized by good flavor, good texture and other favorable organoleptic attributes. Accordingly, the disclosure also encompasses a pet food composition including a mixture of kibble some of which include a sustained-release amount of lipoic acid and other kibble which does not include lipoic acid.

To produce a pet food composition coated with lipoic acid, any technique suitable for coating the pieces may be used. For example, in the case of a liquid carrier substrate, the mixture of the lipoic acid and the carrier substrate may be sprayed onto the dried pieces. This may be carried out in any suitable manner. For example, the pieces may be fed into a fluidized bed onto which the mixture is sprayed. Alternatively, the pieces may be fed into a rotary coater into which the mixture is sprayed. As a further alternative, the pieces may be caused to fall in a curtain and the coating mixture sprayed onto the curtain. Heat sensitive components such as vitamins and amino acids may also be included in the dry mix. The dry mix is then agglomerated on the dried pieces using an agglomerating agent. Fats, oils and sugar solutions are examples of suitable agglomerating agents.

For a pet food composition product filled with lipoic acid, the mixture of the lipoic acid and carrier substrate is filled into the central bore of each piece. In this case, the carrier substrate is preferably viscous or a substance which hardens rapidly. Fats are particularly suitable. Alternatively, the pet food composition may be fed into a tumbler and the carrier substrate agglomerated to the product using a syrup. In this case, the product is coated and filled.

### EXAMPLES

### Reference Example 1

A method for administration of lipoic acid to pets was investigated that would result in a slower, prolonged availability of lipoic acid to companion animals, for example, dogs and cats. Lipoic acid when administered as a capsule has a relatively rapid absorption and disappearance from serum. It is rapidly metabolized by the liver and excreted in the urine. The ability to have a method whereby the lipoic acid is made available over a longer time period but at lower levels may result in increased antioxidant efficacy and reduce potential toxicity to the animal. In addition, it is more convenient for a pet owner to provide the pet with a single food meal containing lipoic acid rather than trying dose capsules multiple times per day or risk harm to their pet by giving a single large dose of lipoic acid.

A study is conducted to evaluate effect of dose and method of delivery on pharmacokinetics of orally administered dl-alpha-lipoic acid (ALA) in 27 healthy dogs. Three different doses of alpha-lipoic acid (2.5, 12.5 or 25 mg/kg bodyweight) and three variations of oral administration are evaluated: 1) lipoic acid capsule form administered after 12 hours of fasting, 2) lipoic acid capsule form given with a food meal (control food), 3) lipoic acid co-manufactured in the process of a complete extruded food meal. The control food without ALA is fed throughout the study period. The analytical results of the foods fed in this study are in Table 1.

**Table 1. Analytical results of foods fed on an as is basis.**

| Nutrient | Control | 2.5 mg/kg ALA | 12.5 mg/kg ALA | 25 mg/kg ALA |
|---|---|---|---|---|
| Moisture (%) | 8.2 | 8.1 | 8.3 | 8.5 |
| Protein (%) | 23.5 | 23.9 | 23.3 | 23.9 |
| Fat (%) | 15.2 | 14.9 | 14.6 | 15.4 |
| Ash (%) | 4.9 | 5.0 | 4.8 | 5.1 |
| Calcium (%) | 0.74 | 0.81 | 0.74 | 0.81 |
| Phosphorus (%) | 0.54 | 0.58 | 0.56 | 0.59 |
| Lipoic Acid (ppm) | 15 | 191 | 602 | 1145 |

A three period Latin square design was used to create a 3x3 factorial arrangement of treatments with dogs assigned to a group following a planned treatment assignment. Serum is collected at 1 minute before alpha-lipoic acid dose and 15, 30, 45, 60, and 120 minutes after a single alpha lipoic acid dosing. Pharmacokinetic parameters are calculated using a non-compartmental model and analyzed using non-parametric tests of WinNonlin 4.1 in SAS v9.]. There was a significant effect of dose for all delivery methods (P<0.05). In addition, there was a significant effect of delivery method (p<0.05) with alpha lipoic acid incorporated in a single dry food meal resulting in lower maximum serum concentrations. Results of the study are in Table

**Table 2: Mean ± Standard Deviation (n = number of animals in mean) for pharmacokinetic parameters (PK parameters) in dogs administered lipoic acid by different methodologies.**

| PK parameters | Administration rate 2.5 mg/kg | | | Administration rate 12.5 mg/kg | | | Administration rate 25 mg/kg | | |
|---|---|---|---|---|---|---|---|---|---|
| | SNF | SF | CMF | SNF | SF | CMF | SNF | SF | CMF |
| Cₘₐₓ (ng/ml) | 529 ± 724 (9) | 206 ± 91 (9) | 47 ± 12(5) | 1696 ± 1847 (9) | 1151 ± 960(9) | 154 ± 59 (7) | 4037 ± 4171 (9) | 5441 ± 3777 (8) | 628 ± 994 (8) |
| AUC_{0-infininty} (min*ng/mL) | 18836 ± 19987 (6) | 8971 ± 1801 (7) | 33878 (1) | 74660 ± 44850 (8) | 59350 ± 19513 (8) | 92215 ± 25869 (4) | 214531 ± 142814 (4) | 218029 ± 100463 (7) | 227304 ± 299998(6) |
| Elimination half life (min) | 19.4 ± 9.7 (6) | 28.6 ± 22.6 (7) | 565 (1) | 29.1 ± 8.4 (8) | 45.1 ± 22.2 (8) | 379.2 ± 137.6 (4) | 46.7 ± 52.5 (4) | 30.2 ± 10 (7) | 573.3 ± 877.8 (6) |

| | | | | | | | | | |
|---|---|---|---|---|---|---|---|---|---|
| SNF = supplement provided as a capsule after fasting; SF=supplement as a capsule fed with food meal; CMF=Co-manufactured supplemental lipoic acid in an extruded dog food matrix. Cmax is the maximal concentration obtained in the plasma after administration of the lipoic acid at different doses. AUC0-infinity is the calculated area under the curve for lipoic acid as administered by the different routes and doses. Elimination half-life is the time to eliminate half the dose of lipoic acid from the plasma space. | | | | | | | | | |

As can be seen from Table 2, the maximal concentration of lipoic acid in plasma is significantly decreased, approximately 8-10 fold, when the substance is co-manufactured in the food thus reducing potential toxicity from higher plasma concentrations. In addition, the elimination half life and the total area under the curve are greater for the co-manufactured food which indicates that the lipoic acid co-manufactured with the food has a slower longer effective time of release to the plasma space.

## Claims

1. A pet food composition in the form of a kibble comprising a gelatinized starch matrix and lipoic acid or a salt thereof, which has a sustained-release coating, wherein the sustained-release coating is selected from carnauba wax, spermaceti wax, candellila wax, cocoa butter, cetostearyl alcohol, beeswax, partially hydrogenated vegetable oils, ceresin, paraffin, myristyl alcohol, stearyl alcohol, cetyl alcohol, stearic acid, acacia gum, gelatin, tragacanth, veegum, xanthan gum, carboxymethyl cellulose, hydroxypropyl cellulose, and hydroxyethyl cellulose.

2. The pet food composition of claim 1, wherein the lipoic acid is incorporated into matrix sphereoids.

3. The pet food composition of claim 1, wherein the lipoic acid is incorporated into a normal-release core.

4. The pet food composition of claim 1, wherein the lipoic acid is incorporated into a core which comprises a matrix including a sustained-release material.

5. The pet food composition of any preceding claim, which is additionally coated with one or more materials suitable for the regulation of release or for the protection of the formulation.

6. The pet food composition of any preceding claim, wherein the coating permits pH-independent release.

7. The pet food composition of claims 1 to 5, wherein the coating permits pH-dependent release.

8. The pet food composition of any preceding claim which is a cat food or a dog food.

9. The pet food composition of claim 8, which is a cat food and which comprises lipoic acid in an amount in the range 65 ppm to 2600 ppm.

10. The pet food composition of claim 8, which is a dog food and which comprises lipoic acid in an amount in the range 50 ppm to 4500 ppm.

11. The pet food composition of any preceding claim, wherein the sustained-release material is hydroxypropyl cellulose and wherein the sustained-release material is present in the composition in an amount of 4% to 20% by weight.

12. The pet food composition of any preceding claim, for use in preventing cell damage in a companion animal.

## Patentansprüche

1. Haustierfutterzusammensetzung in Form eines Pellets umfassend eine gelatinisierte Stärkematrix und Liponsäure oder eines ihrer Salze, welches einen Überzug zur verzögerten Freisetzung umfasst, wobei der Überzug zur verzögerten Freisetzung ausgewählt ist aus Carnaubawachs, Spermaceti, Candelillawachs, Kakaobutter, Cetylstearylalkohol, Bienenwachs, partiell hydrogenierten Pflanzenölen, Ceresin, Paraffin, Myristylalkohol, Stearylalhohol, Cetylalkohol, Stearinsäure, Gummi arabicum, Gelatine, Traganth, Veegum, Xanthangummi, Carboxymethylzellulose, Hydroxypropylzellulose und Hydroxyethylzellulose.

2. Haustierfutterzusammensetzung nach Anspruch 1, wobei die Liponsäure in Matrix-Spheroiden inkorporiert ist.

3. Haustierfutterzusammensetzung nach Anspruch 1, wobei die Liponsäure in einem Kern mit normaler Freisetzung inkorporiert ist.

4. Haustierfutterzusammensetzung nach Anspruch 1, wobei die Liponsäure in einem Kern inkorporiert ist, der eine Matrix umfasst, die ein Material zur verzögerten Freisetzung enthält.

5. Haustierfutterzusammensetzung nach einem der vorangehenden Ansprüche, welche zusätzlich mit einem oder mehreren Materialien beschichtet ist, die zur Regulation der Freisetzung oder zum Schutz der Formulierung geeignet sind.

6. Haustierfutterzusammensetzung nach einem der vorangehenden Ansprüche, wobei die Beschichtung eine pH-unabhängige Freisetzung erlaubt.

7. Haustierfutterzusammensetzung nach Ansprüchen 1 bis 5, wobei die Beschichtung eine pH-abhängige Freisetzung erlaubt.

8. Haustierfutterzusammensetzung nach einem der vorangehenden Ansprüche, welche Katzenfutter oder Hundefutter ist.

9. Haustierfutterzusammensetzung nach Anspruch 8, welche Katzenfutter ist und welche Liponsäure in einer Menge in dem Bereich von 65 ppm bis 2600 ppm umfasst.

10. Haustierfutterzusammensetzung nach Anspruch 8, welche Hundefutter ist und welche Liponsäure in einer Menge in dem Bereich von 50 ppm bis 4500 ppm umfasst.

11. Haustierfutterzusammensetzung nach einem der vorangehenden Ansprüche, wobei das Material zur verzögerten Freisetzung Hydroxypropylzellulose ist und wobei das Material zur verzögerten Freisetzung in der Zusammensetzung in einer Menge von 4% bis 20 %, bezogen auf das Gewicht, vorliegt.

12. Haustierfutterzusammensetzung nach einem der vorangehenden Ansprüche zur Verwendung in der Vermeidung von Zellschäden bei einem Haustier.

## Revendications

1. Composition alimentaire pour animaux de compagnie sous la forme d'une croquette comprenant une matrice d'amidon gélatinisé et de l'acide lipoïque ou un sel de celui-ci, qui a un revêtement à libération prolongée, dans lequel le revêtement à libération prolongée est choisi parmi la cire de carnauba, la cire de spermaceti, la cire de candellila, le beurre de cacao, l'alcool cétostéarylique, la cire d'abeille, les huiles végétales partiellement hydrogénées, la cérésine, la paraffine, l'alcool myristylique, l'alcool stéarylique, l'alcool cétylique, l'acide stéarique, la gomme arabique, la gélatine, la gomme adragante, le Veegum, la gomme xanthane, la carboxyméthylcellulose, l'hydroxypropylcellulose et l'hydroxyéthylcellulose.

2. Composition alimentaire pour animaux de compagnie selon la revendication 1, dans laquelle l'acide lipoïque est incorporé dans des sphéroïdes matrices.

3. Composition alimentaire pour animaux de compagnie selon la revendication 1, dans laquelle l'acide lipoïque est incorporé dans un noyau à libération normale.

4. Composition alimentaire pour animaux de compagnie selon la revendication 1, dans laquelle l'acide lipoïque est incorporé dans un noyau qui comprend une matrice comprenant un matériau à libération prolongée.

5. Composition alimentaire pour animaux de compagnie selon l'une quelconque des revendications précédentes, qui est en outre revêtue d'un ou plusieurs matériaux appropriés pour la régulation de la libération ou pour la protection de la formulation.

6. Composition alimentaire pour animaux de compagnie selon l'une quelconque des revendications précédentes, dans laquelle le revêtement permet une libération indépendante du pH.

7. Composition alimentaire pour animaux de compagnie selon les revendications 1 à 5, dans laquelle le revêtement permet une libération dépendante du pH.

8. Composition de nourriture pour animaux de compagnie selon l'une quelconque des revendications précédentes, qui est un aliment pour chats ou un aliment pour chiens.

9. Composition alimentaire pour animaux de compagnie selon la revendication 8, qui est un aliment pour chats et qui comprend de l'acide lipoïque en une quantité dans la plage de 65 ppm à 2 600 ppm.

10. Composition alimentaire pour animaux de compagnie selon la revendication 8, qui est un aliment pour chiens et qui comprend de l'acide lipoïque en une quantité dans la plage de 50 ppm à 4 500 ppm.

11. Composition alimentaire pour animaux de compagnie selon l'une quelconque des revendications précédentes, dans laquelle le matériau à libération prolongée est l'hydroxypropylcellulose et dans laquelle le matériau à libération prolongée est présent dans la composition en une quantité de 4 % à 20 % en poids.

12. Composition alimentaire pour animaux de compagnie selon l'une quelconque des revendications précédentes, pour une utilisation dans la prévention de lésions cellulaires chez un animal de compagnie.
